(19) Europäisches Patentamt
European Patent Office
Office européen des brevets

(11) Veröffentlichungsnummer: **0 158 987 B1**

# (12) EUROPÄISCHE PATENTSCHRIFT

(45) Veröffentlichungstag der Patentschrift: **31.01.90**

(51) Int. Cl.⁴: **C 07 K 7/20**, A 61 K 37/02

(21) Anmeldenummer: **85104446.1**

(22) Anmeldetag: **12.04.85**

## (54) Verwendung von Gonadoliberin und Gonadoliberinagonisten zur Herstellung einer pharmazeutischen Zubereitung.

(30) Priorität: **18.04.84 DE 3414595**

(43) Veröffentlichungstag der Anmeldung: **23.10.85 Patentblatt 85/43**

(45) Bekanntmachung des Hinweises auf die Patenterteilung: **31.01.90 Patentblatt 90/5**

(84) Benannte Vertragsstaaten: **AT BE CH DE FR GB IT LI LU NL SE**

(56) Entgegenhaltungen:
**EP-A- 0 137 294**
**FR-A- 2 450 109**
**FR-A- 2 465 486**

**Pschyrembel Klinisches Wörterbuch, 255 Auflage 1986, S. 389**
**Med. Klinik 82 (1987) 135-139**
**Klin. Wochenschrift 62(1984), 129-132**
**Med. Praxis 79(1984) Nr. 24, S. 20-26**
**Calcified Tissues 41(1987) 20-21**

(73) Patentinhaber: **HOECHST AKTIENGESELLSCHAFT, Postfach 80 03 20, D-6230 Frankfurt am Main 80 (DE)**

(72) Erfinder: **König, Wolfgang, Dr., Eppsteiner Strasse 25, D-66238 Hofheim am Taunus (DE)**

**EP 0 158 987 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Es wurde gefunden, daß Gonadoliberin und dessen agonistisch wirkenden Analoga überraschenderweise in geringer Dosierung sowohl bei klimakterischen Beschwerden in der Prämenopause als auch in der Postmenopause einen positiven Effekt ausüben.

Die Erfindung betrifft daher die Verwendung von Gonadoliberin oder dessen mindestens ebenso stark wirksamen Agonisten zur Herstellung einer pharmazeutischen Zubereitung zur Behandlung von klimakterischen Beschwerden und pathologischen Zuständen mit zu hohem Parathormonspiegel, gekennzeichnet durch einen pharmakologisch wirksamen Gehalt an einer solchen Verbindung in einem pharmazeutisch unbedenklichen Träger.

Bei Osteoporose findet man im Knochenmark erhöhte Parathormon-Spiegel (Klin. Wochenschr. 62, 129–132, 1984); Parathormon wirkt knochenauflösend. Bei jungen weiblichen Ratten mit hohem Parathormon-Spiegel erniedrigten kleine Dosen des Gonadoliberinagonisten Buserelin Plasma-Parathormon, obwohl ein durch Kalzium-Infusion erniedrigter Parathormon-Spiegel durch ähnlich dosiertes Buserelin erhöht wird (vgl. deutsche Patentanmeldung P 3 332 329.1). Möglicherweise spielt diese Parathormon-regulierende Wirkung des Buserelins eine Rolle für die Behandlung von Knochenschmerzen und der Osteoporose.

Die Erfindung betrifft deshalb auch die Verwendung von Gonadoliberin oder dessen mindestens ebenso stark wirksamen Agonisten zur Herstellung einer pharmazeutischen Zubereitung zur Behandlung der Osteoporose, gekennzeichnet durch einen pharmakologisch wirksamen Gehalt einer solchen Verbindung in einem pharmazeutisch unbedenklichen Träger.

Weiterhin ist auch bei der Niereninsuffizienz (Akt. Endokr. Stoffw. 5 [1984] 180–186) der Parathormonspiegel erhöht.

Deshalb betrifft die Erfindung auch die Verwendung von Gonadoliberin oder dessen mindestens ebenso stark wirksamen Agonisten zur Herstellung einer pharmazeutischen Zubereitung zur Behandlung der Niereninsuffizienz, gekennzeichnet durch einen pharmakologisch wirksamen Gehalt einer solchen Verbindung in einem pharmazeutisch unbedenklichen Träger.

Beschwerden, die mit Gonadoliberin oder dessen agonistischen Analoga behandelt werden können, sind demnach Zyklusstörungen (unregelmäßige Regelblutungen) in der Prämenopause, die postklimakterischen Beschwerden, wie z.B. Hitzewallungen, Kälteschauer, Schweißausbrüche, Schwindel, Herzklopfen, Schlaflosigkeit, Angstgefühle, Depressionen, Kopfschmerzen, Knochenschmerzen, die Osteoporose und die Niereninsuffizienz.

Gonadoliberin ist ein Dekapeptid der Formel I, das im Hypothalamus gebildet wird und in der Hypophyse die Freisetzung von Follitropin und Lutropin bewirkt (Biochem. Biophys. Res. Commun. 43, 1971, Seite 1334).

Glu-His-Trp-Ser-Tyr-Gly-Leu-Arg-Pro-Gly-$NH_2$ (I)

Die diagnostische Dosis von Gonadoliberin zur Freisetzung der Gonadotropine (Follitropin und Lutropin) liegt bei einer parenteralen Applikation von 25–100 µg/erwachsener Mensch (Rote Liste 1983, 34 002 und 24 004). Die bei Kryptorchismus verwendete intranasale Dosierung bei Kindern zwischen zwei und sechs Jahren beträgt 3 mal täglich 400 µg (Rote Liste 1983, 49 038).

Dagegen genügen bei der Behandlung von klimakterischen Beschwerden und pathologischen Zuständen mit zu hohem Parathormonspiegel, sowie bei der Behandlung von Osteoporose und Niereninsuffizienz parenterale Gaben von nur 0,5 bis 5 µg Gonadoliberin/Einzeldosis für einen normalgewichtigen Erwachsenen (= 6 bis 60 ng/kg/Dosierung) oder die gleich stark wirksame Menge eines Gonadoliberinagonisten. Bei intranasaler Applikation werden wegen der schlechten Resorption etwa 10 bis 200 µg Gonadoliberin/Einzeldosis für einen normalgewichtigen Erwachsenen oder die gleich stark wirksame Menge eines Gonadoliberinagonisten appliziert. Im allgemeinen dosiert man am Anfang der Behandlung 2–3 mal täglich. Nachdem die Beschwerden nachgelassen haben, kann man sich auf eine einmalige Applikation/Tag beschränken.

Die agonistische Wirkung des Gonadoliberins wird vor allem durch die Substitution von Gly-$NH_2^{10}$ mit dem Ethylamin-Rest und durch den Austausch von $Gly^6$ mit lipophilen D-Aminosäuren verstärkt. Man kommt so zu Verbindungen, die je nach Test etwa 100–200 mal stärker als Gonadoliberin wirken (Vitamins and Hormones 38, 1980, Seite 257–323). Wichtig für den Austausch in Position 6 ist, daß die D-Aminosäure eine α-CH- und eine β-$CH_2$-Gruppe enthält (Peptides–Chemistry, Structure, Biology, Seite 883–888, Ann Arbor Science Publishers Inc Ann Arbor, Mich. 1975). Von den «natürlichen» D-Aminosäuren bewährten sich am besten D-Trp, D-Leu und der tert.-Butylether von D-Ser. Durch die Verwendung synthetischer aromatischer D-Aminosäuren lassen sich jedoch noch höhere Aktivitäten erzielen, wie z.B. bei [D-3-(2,4,6.-Trimethylphenyl)-$Ala^6$]- und [D-3-(2-Naphthyl)-$Ala^6$] Gonadoliberin (J. Med. Chem. 25, 1982 Seite 795–801).

Infrage für die Indikation kommen alle Gonadoliberinanaloga mit voller oder stärkerer Gonadoliberinwirkung. Geeignet sind beispielsweise folgende Analoga der Formel II:

Glu–His–Trp–Ser–Tyr–X–Y–Arg–Pro–Z (II)
1 2 3 4 5 6 7 8 9 10

in welcher bedeutet (Abkürzungen siehe Houben-Weyl, Methoden der organischen Chemie, 4, Auflage., Bd. XV/1 und XV/2, Thieme Verlag Stuttgart):

a) Z = Gly-$NH_2$
Y = Leu und
X = D-Nle, D-Nva, D-Abu, D-Phe, D-Ser, D-Met, D-Pgl, D-Lys, D-Leu, D-Arg, D-Ser($Bu^t$), D-Thr($Bu^t$), D-Cys($Bu^t$), D-Lys, D-Asp, D-Asp($OBu^t$), D-Glu($OBu^t$), D-Orn(Boc), D-Lys(Boc), D-Trp, D-Tyr, ε-Lauryl-D-Lys oder ε-Dextran-D-Lys, D-His(Bzl)

oder

b) Z = Gly-$NH_2$, NH-($C_1$–$C_3$)-Alkyl bzw. NH-Cyclopropyl, die durch OH oder F substituiert sein können,
Y = Leu, Ser($Bu^t$), Cys($Bu^t$), Glu($OBu^t$), Orn-(Boc) oder Lys(Boc) und
X = D-Ser($Bu^t$), D-Cys($Bu^t$), D-Asp($OBu^t$), D-Glu($OBu^t$), D-Orn(Boc) oder D-Lys(Boc), D-His(Bzl)

wobei $Ser^4$ gegebenenfalls durch Ala oder Thr, Tyr gegebenenfalls durch Phe und Arg gegebenenfalls durch Orn, Lys oder Homoarginin ersetzt ist,

oder

c) Z = $-NHCH_3$, $-NH-CH_2-CH_3$, $-NH-CH_2CH_2CH_3$,

$-NHCH_2CH_2-OH$, 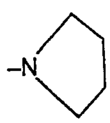 oder 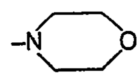 ,

Y = Leu und
X = Gly

oder

d) Z = $-NHC_2H_5$
Y = Leu und
X = D-Trp, D-Leu, D-Ala, D-Ser($Bu^t$), D-Tyr, D-Lys oder D-His(Bzl)

oder

e) Z = Gly-$NH_2$ oder NH-$C_2H_5$,
Y = N-α-Methyl-Leu und
X = Gly

oder

f) Z = NH-Cyclopropyl,
Y = Leu und
X = D-Leu

oder

g) Z = Gly-$NH_2$, NH-($C_1$–$C_3$)-Alkyl oder NH-Cyclopropyl,
Y = Ser($Bu^t$), Cys($Bu^t$), Asp($OBu^t$), Glu($OBu^t$), Orn(Boc) oder Lys(Boc) und
X = Gly.

Verbindungen der Formel II sowie Verfahren zu deren Herstellung sind beispielsweise bekannt aus:

– Coy D.H., Labrie F., Favary M., Coy E.J. et Schally A.V. (1975) LH – releasing activity of patent LH – RH analogs in vitro BBRC 67, 576–582.

– Wylie Vale et al. (1976) in «Hypothalamus and endocrine functions» (F. Labrie, J. Meites and G. Pelletier, eds; Plenum Press) pages 397–429.

Fujino, BBRC vol. 49, n° 3, 1972, page 863.

DE-OS 2 509 783, US-PS 3 901 872, US-PS 3 896 104, JP-PS 4 9100–081, US-PS 3 971 737, BE-PS 842–857, BE-PS 832–310, BE-PS 832–311, US-PS 4 003 884, US-PS 4 024 248, DE-OS 2 720 245 und DE-OS 2 446 005.

Als besonders geeignet haben sich beispielsweise folgende Analoga erwiesen:

[D-Ser($Bu^t$)$^6$] Gonadoliberin-(1-9)-nonapeptid-ethylamid (= Buserelin)
(Drugs of the Future 4, 1979, Seite 173–177, 8, 1983 S. 254),
[D-Trp$^6$] Gonadoliberin (Drugs of the Future 3, 1978, 2. 645–646),
[D-Trp$^6$] Gonadoliberin-(1-9)-nonapeptid-ethylamid (Drugs of the Future 7, 1982, Seite 637–642),
[D-Leu$^6$] Gonadoliberin-(1-9)-nonapeptid-ethylamid (Drugs of the Future 7, 1982, Seite 882–886),
[D-Ser($Bu^t$)$^6$, AzaGly$^{10}$] Gonadoliberin (Drugs of the Future 5, 1980, Seite 191–192; 1983, Seite 364–365),
[D-Trp$^6$, N-MeLeu$^7$] Gonadoliberin-(1-9)-nonapeptidethylamid (Drugs of the Future 8, 1983, Seite 347–350),
[D-α-Aminodipinsäure-δ-tert.butylester$^6$] Gonadoliberin-(1-9)-nonapeptid-ethylamid (DE-OS 3 020 941) und die oben einleitend aufgezählten Analoga mit den unnatürlichen D-Aminosäuren.

Die erfindungsgemäßen Zubereitungen können intranasal oder parenteral verabreicht werden. Für eine intranasale Anwendungsform wird die Verbindung mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Als ölige Trägerstoffe oder Lösungsmittel kommen beispielsweise pflanzliche oder tierische Öle in Betracht, wie Sonnenblumenöl oder Lebertran.

Zur subkutanen oder intravenösen Applikation wird die aktive Verbindung oder deren physiologisch verträgliche Salze, gewünschtenfalls mit den dafür üblichen Substanzen wie Lösungsvermittler, Emulgatoren oder weitere Hilfsstoffe in Lösung, Suspension oder Emulsion gebracht.

Als Lösungsmittel kommen z.B. in Frage: Wasser, physiologische Kochsalzlösungen oder Alkohole, z.B. Ethanol, Propandiol oder Glycerin daneben auch Zuckerlösungen wie Glukose- oder Mannitlösungen, oder auch eine Mischung aus den verschiedenen genannten Lösungsmitteln.

Die hier im einzelnen aufgezählten LH-RH-Analoga sind alle hochwirksam. Sie wirken am Menschen etwa 10 mal stärker als Gonadoliberin und haben eine längere Wirkungsdauer. Sie müssen also in geringer Dosierung und nicht so oft wie Gonadoliberin dosiert werden. Gewöhnlich genügen bei parenteraler Gabe 50 bis 500 ng der o.g. Gonadoliberin-agonisten/normalgewichtigen Frau (= 0,6 bis 70 ng/kg/Dosis). Bei Applikation auf die Schleimhäute (z.B. intranasale Gabe) werden 1–20 µg Analogen/normalgewichtigen Erwachsenen (= 10 bis 300 ng/kg/Dosis) notwendig. Es kann jedoch auch notwendig werden, bei besonders stark wirksamen LH-RH-Analoga, die Dosis zu erniedrigen, bzw. bei geringer wirksamen, diese zu erhöhen.

Beispiel 1 (Zubereitung zur intranasalen Anwendung):

4,0 g ⌐Glu-His-Trp-Ser-Tyr-D-Ser(Bu$^t$)-Leu-Arg-Pro-NH-$C_2H_5$-diacetat werden in 100 ml destilliertes Wasser gelöst. Gleichzeitig löst man 31,2 g $NaH_2PO_4 \cdot 2H_2O$, 66,29 g $Na_2HPO_4$, 25 g NaCl und 100 g Benzylalkohol in 8 l dest. Wasser und gibt 500 g Polyvinylalkohol mit einem K-Wert von ca. 90 zu. Die beiden Lösungen werden vereinigt und filtriert.

Beispiel 2 (Zubereitung zur intranasalen Anwendung):

100 g wasserfreies Lanolin und 440 g Vaseline werden zusammengeschmolzen. Zu der erkalteten Schmelze gibt man eine Suspension von 800 mg mikrofeinem ⌐Glu-His-Trp-Ser-Phe-D-Glu(OBu$^t$)-Leu-Arg-Pro-NH-$C_2H_5$-diacetat in 359,2 g flüssigem Paraffin. Zum Schluß werden 10 g Benzylalkohol dazugegeben und die Salbe homogenisiert.

Beispiel 3 (Zubereitung für Injektionen):

Man löst 2 mg ⌐Glu-His-Trp-Ser-Tyr-D-Lys-(Boc)-Leu-Arg-Pro-NH-$C_2H_5$-diacetat in 500 ml bidestilliertes Wasser und versetzt mit 100 ml Phosphatpuffer pH 4,5. Dann gibt man 1 g Mannit und die errechnete Menge NaCl zur Isotonie zu und füllt mit Wasser auf 1 Liter auf. Nach Sterilfiltration wird in Ampullen zu 1 bzw. 2 ml abgefüllt und lyophilisiert.

Beispiel 4 (Zubereitung für Injektionen):

Man arbeitet nach Beispiel 3, gibt aber vor dem Auffüllen mit Wasser 2,5 g 4-Hydroxybenzoesäuremethylester zu. Nach Sterilfiltration wird in Ampullen zu 1 oder 2 ml abgefüllt.

Beispiel 5

Buserelin wird zweckmäßig intranasal in einer Dosis von 1 bis 10 µg/normalgewichtige Frau appliziert. Man dosiert bis zum Verschwinden der Beschwerden einmal am Tag. Anschließend kann man die Therapie mit einer Applikation an jedem zweiten Tag fortsetzen.

Diese Therapie kann auch bei den Beschwerden des Klimakterium virile des Mannes angewendet werden.

Beispiel 6

Parathormon-senkende Wirkung von Buserelin (siehe Fig.)

4 Gruppen von je 5 weiblichen Wistarratten wurden mit Placebo bzw. drei verschiedenen Dosen (1 ng/kg, 5 ng/kg, 50 ng/kg) Buserelin behandelt. Zunächst entnahm man von allen Tieren eine Blutprobe (Ausgangswert). Danach gab man den Tieren eine Injektion von Buserelin in physiologischer Kochsalzlösung mit 0,25% Haemaccel$^R$-Zusatz und der Placebogruppe die gleiche Menge physiologischer Kochsalzlösung mit 0,25% Haemaccel$^R$-Zusatz. Nach 90 Minuten und 240 Minuten wurden Blutproben entnommen. Das Blutserum wurde auf Parathormon untersucht. Nach 90 Minuten fiel in der Verum-Gruppe der Parathormon-Spiegel ab, wobei der beste Effekt bei den kleinen Dosierungen zu beobachten war. Nach 240 Minuten war der Ausgangswert wieder erreicht.

Erläuterung zur Fig.: Es ist der zeitliche Verlauf des PTH-Spiegels nach i.v.-Buserelin-Gabe von 1 ng/kg (Kurve 1), 5 ng/kg (Kurve 2) und 50 ng/kg (Kurve 3) wiedergegeben. Kurve 4 zeigt den Verlauf bei Placebo-Gabe. Die Meßwerte sind jeweils mit Standardabweichung angegeben (n = 5).

Beispiel 7

Eine Frau (47 Jahre) mit unregelmäßiger Menstruation und starken krampfartigen Schmerzen während der Periode, sowie einem dauernden Druck auf der Blase und den typischen Hitzewallungen wurde mit 100 µg (intranasal) Gonadoliberin zwei mal täglich (am Morgen und Abend) therapiert. Schon nach dem 1. Tag der Therapie waren die Beschwerden verschwunden. Die Patientin wurde etwa 9 Monate behandelt. Die Regelblutungen kamen in dieser Zeit sehr pünktlich.

Die Therapie wurde zwei mal für je zwei Wochen ausgesetzt. Dabei stellten sich jedoch oben genannte Beschwerden wieder ein, so daß nach dem beschriebenen Schema weiter therapiert wurde. Negative Nebenwirkungen des Gonadoliberins wurden nicht beobachtet.

Beispiel 8

Eine Frau (35 Jahre) bekam nach operativer Entfernung des Uterus starke Migräne-Anfälle und Knochenschmerzen. Beide Symptome konnten durch eine Behandlung mit 2 mal täglich 50 µg Gonadoliberin (intranasal) gebessert werden. Die Knochenschmerzen wurden auf ein Minimum reduziert und die Migräne-Anfälle verliefen kürzer, seltener und wesentlich schwächer als ohne Gonadoliberin-Therapie.

**Patentansprüche**

1. Verwendung von Gonadoliberin oder dessen mindestens ebenso stark wirksamen Agonisten zur Herstellung einer pharmazeutischen Zubereitung zur Behandlung von klimakterischen Beschwerden und pathologischen Zuständen mit zu hohem Parathormonspiegel, gekennzeichnet durch einen pharmakologisch wirksamen Gehalt an einer solchen Verbindung in einem pharmazeutisch unbedenklichen Träger.

2. Verwendung gemäß Anspruch 1 zur Herstellung einer Zubereitung gemäß Anspruch 1 zur parenteralen Verabreichung, gekennzeichnet durch einen Gehalt an 0,5 bis 5 µg Gonadoliberin/Einzeldosis für einen normalgewichtigen Erwachsenen oder die gleich stark wirksame Menge eines Gonadoliberinagonisten.

3. Verwendung gemäß Anspruch 1 zur Herstellung einer Zubereitung zur Verabreichung auf die Schleimhaut, gekennzeichnet durch einen Gehalt an 10 bis 200 µg Gonadoliberin/Einzeldosis für einen normalgewichtigen Erwachsenen oder die gleich stark wirksame Menge eines Gonadoliberinagonisten.

4. Verwendung von Gonadoliberin oder dessen mindestens ebenso stark wirksamen Agonisten

zur Herstellung einer pharmazeutischen Zubereitung zur Behandlung der Osteoporose, gekennzeichnet durch einen pharmakologisch wirksamen Gehalt einer solchen Verbindung in einem pharmazeutisch unbedenklichen Träger.

5. Verwendung gemäß Anspruch 4 zur Herstellung einer Zubereitung gemäß Anspruch 4 zur parenteralen Verabreichung, gekennzeichnet durch einen Gehalt an 0,5 bis 5 µg Gonadoliberin/Einzeldosis für einen normalgewichtigen Erwachsenen oder die gleich stark wirksame Menge eines Gonadoliberinagonisten.

6. Verwendung gemäß Anspruch 4 zur Herstellung einer Zubereitung gemäß Anspruch 4 zur Verabreichung auf die Schleimhaut, gekennzeichnet durch einen Gehalt an 10 bis 200 µg Gonadoliberin/Einzeldosis für einen normalgewichtigen Erwachsenen oder die gleich stark wirksame Menge eines Gonadoliberinagonisten.

7. Verwendung von Gonadoliberin oder dessen mindestens ebenso stark wirksamen Agonisten zur Herstellung einer pharmazeutischen Zubereitung zur Behandlung der Niereninsuffizienz, gekennzeichnet durch einen pharmakologisch wirksamen Gehalt einer solchen Verbindung in einem pharmazeutisch unbedenklichen Träger.

8. Verwendung gemäß Anspruch 7 zur Herstellung einer Zubereitung gemäß Anspruch 7 zur parenteralen Verabreichung, gekennzeichnet durch einen Gehalt an 0,5 bis 5 µg Gonadoliberin/Einzeldosis für einen normalgewichtigen Erwachsenen oder die gleich stark wirksame Menge eines Gonadoliberinagonisten.

9. Verwendung gemäß Anspruch 7 zur Herstellung einer Zubereitung gemäß Anspruch 7 zur Verabreichung auf die Schleimhaut, gekennzeichnet durch einen Gehalt an 10 bis 200 µg Gonadoliberin/Einzeldosis für einen normalgewichtigen Erwachsenen oder die gleich stark wirksame Menge eines Gonadoliberinagonisten.

10. Verfahren zur Herstellung einer Zubereitung gemäß einem der Ansprüche 1 bis 9, dadurch gekennzeichnet, daß man Gonadoliberin oder dessen Analogon zusammen mit einem pharmazeutisch unbedenklichen Träger in eine geeignete Zubereitungsform bringt.

## Claims

1. The use of gonadoliberin or of its agonists which are at least as active for preparing a pharmaceutical composition for the treatment of climacteric complaints and pathological states in which the level of parathyroid hormone is too high, which has a pharmacologically active content of a compound of this type in a pharmaceutically acceptable vehicle.

2. The use as claimed in claim 1 for preparing a composition as claimed in claim 1 for parenteral administration, which contains 0.5 to 5 µg of gonadoliberin/single dose for an adult of normal weight, or the equally active amount of a gonadoliberin agonist.

3. The use as claimed in claim 1 for preparing a composition for administration onto the mucosa, which contains 10 to 200 µg of gonadoliberin/single dose for an adult of normal weight, or the equally active amount of a gonadoliberin agonist.

4. The use of gonadoliberin or of its agonists which are at least as active for preparing a pharmaceutical composition for the treatment of osteoporosis, which has a pharmacologically active content of a compound of this type in a pharmaceutically acceptable vehicle.

5. The use as claimed in claim 4 for preparing a composition as claimed in claim 4 for parenteral administration, which contains 0.5 to 5 µg of gonadoliberin/single dose for an adult of normal weight, or the equally active amount of a gonadoliberin agonist.

6. The use as claimed in claim 4 for preparing a composition as claimed in claim 4 for administration onto the mucosa, which contains 10 to 200 µg of gonadoliberin/single dose for an adult of normal weight, or the equally active amount of a gonadoliberin agonist.

7. The use of gonadoliberin or of its agonists which are at least as active for preparing a pharmaceutical composition for the treatment of renal insufficiency, which has a pharmacologically active content of a compound of this type in a pharmaceutically acceptable vehicle.

8. The use as claimed in claim 7 for preparing a composition as claimed in claim 7 for parenteral administration, which contains 0.5 to 5 µg of gonadoliberin/single dose for an adult of normal weight, or the equally active amount of a gonadoliberin agonist.

9. The use as claimed in claim 7 for preparing a composition as claimed in claim 7 for administration onto the mucosa, which contains 10 to 200 µg of gonadoliberin/single dose for an adult of normal weight, or the equally active amount of a gonadoliberin agonist.

10. A process for preparing a composition as claimed in one of claims 1 to 9, which comprises converting gonadoliberin or its analog together with a pharmaceutically acceptable vehicle into a suitable formulation.

## Revendications

1. Utilisation de la gonadolibérine ou de ses agonistes au moins aussi actifs, pour la préparation d'une composition pharmaceutique destinée au traitement de troubles de la ménopause et d'états pathologiques à taux trop élevés de parathormone, caractérisée par une teneur pharmacologiquement active en un tel composé, dans un véhicule pharmaceutiquement acceptable.

2. Utilisation selon la revendication 1, pour la préparation d'une composition selon la revendication 1 pour administration parentérale, caractérisée par une teneur de 0,5 à 5 µg en gonadolibérine par dose unitaire pour un adulte de poids normal, ou par la quantité aussi active d'un agoniste de gonadolibérine.

3. Utilisation selon la revendication 1, pour la préparation d'une composition pour administration sur la muqueuse, caractérisée par une teneur

de 10 à 200 µg en gonadolibérine par dose unitaire pour un adulte de poids normal ou par la quantité aussi active d'un agoniste de gonadolibérine.

4. Utilisation de la gonadolibérine ou de ses agonistes au moins aussi actifs, pour la préparation d'une composition pharmaceutique destinée au traitement de l'ostéoporose, caractérisée par une teneur pharmacologiquement active en un tel composé, dans un véhicule pharmaceutiquement acceptable.

5. Utilisation selon la revendication 4, pour la préparation d'une composition selon la revendication 4 pour administration parentérale, caractérisée par une teneur de 0,5 à 5 µg en gonadolibérine par dose unitaire pour un adulte de poids normal, ou par la quantité aussi active d'un agoniste de gonadolibérine.

6. Utilisation selon la revendication 4, pour la préparation d'une composition selon la revendication 4 pour administration sur la muqueuse, caractérisée par une teneur de 10 à 200 µg en gonadolibérine par dose unitaire pour un adulte de poids normal ou par la quantité aussi active d'un agoniste de gonadolibérine.

7. Utilisation de la gonadolibérine ou de ses agonistes au moins aussi actifs, pour la préparation d'une composition pharmaceutique destinée au traitement de l'insuffisance rénale, caractérisée par une teneur pharmacologiquement active en un tel composé, dans un véhicule pharmaceutiquement acceptable.

8. Utilisation selon la revendication 7, pour la préparation d'une composition selon la revendication 7 pour administration parentérale, caractérisée par une teneur de 0,5 à 5 µg en gonadolibérine par dose unitaire pour un adulte de poids normal, ou par la quantité aussi active d'un agoniste de gonadolibérine.

9. Utilisation selon la revendication 7, pour la préparation d'une composition selon la revendication 7, pour administration sur la muqueuse, caractérisée par une teneur de 10 à 200 µg en gonadolibérine par dose unitaire pour un adulte de poids normal ou par la quantité aussi active d'un agoniste de gonadolibérine.

10. Procédé pour la préparation d'une composition selon l'une des revendications 1 à 9, caractérisé en ce que l'on met sous une forme pharmaceutique appropriée de la gonadolibérine ou un de ses analogues, conjointement avec un véhicule pharmaceutiquement acceptable.

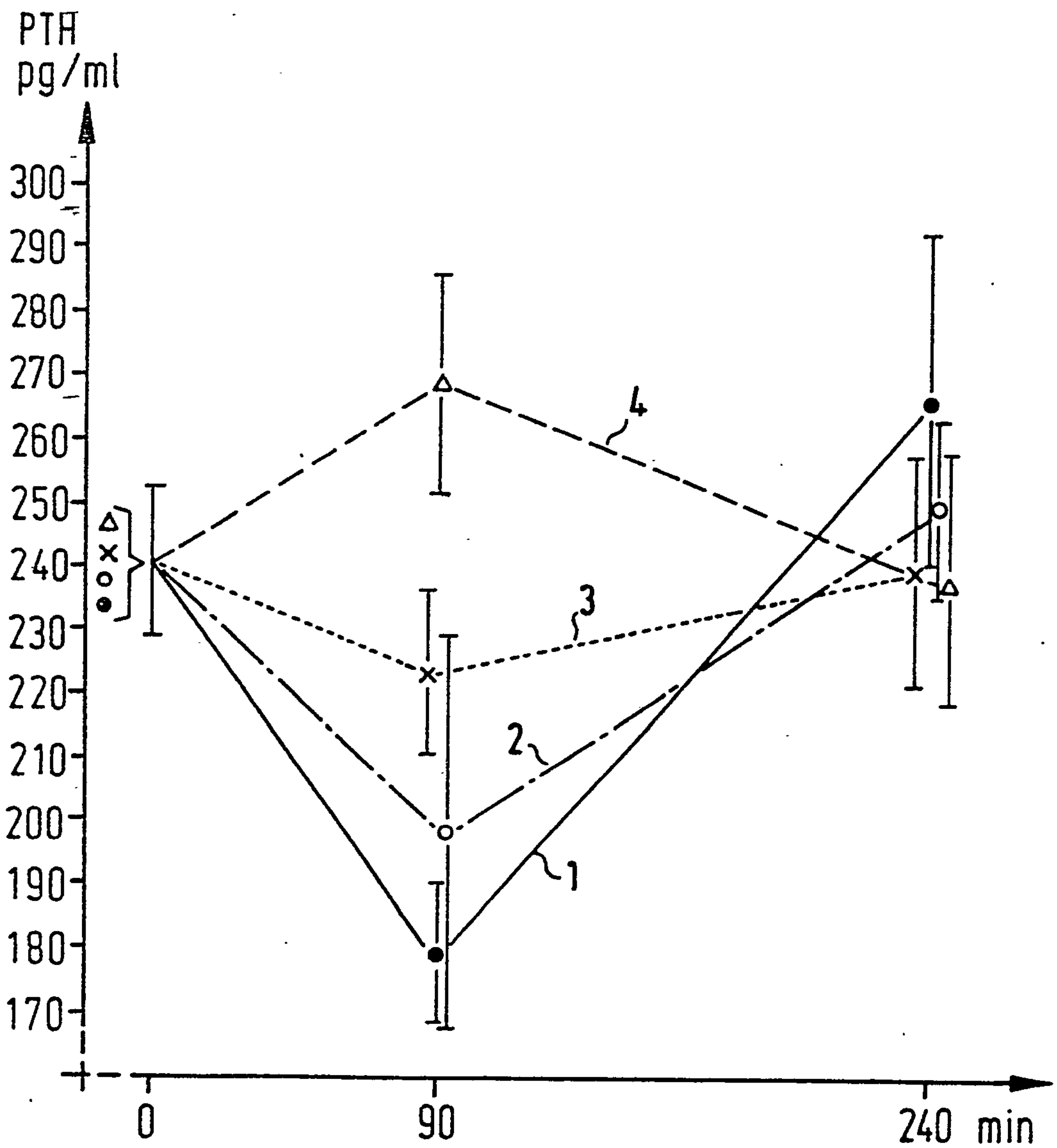
PTH
pg/ml
300
290
280
270
260
250
240
230
220
210
200
190
180
170
0
90
240 min
1
2
3
4